Europäisches Patentamt

European Patent Office

Office européen des brevets

(11) Veröffentlichungsnummer: **0 253 984**
**A1**

## (12) EUROPÄISCHE PATENTANMELDUNG

(21) Anmeldenummer: 87107316.9

(22) Anmeldetag: 20.05.87

(51) Int. Cl.³: **G 01 C 22/02**

(30) Priorität: 24.05.86 DE 3617591

(43) Veröffentlichungstag der Anmeldung:
27.01.88 Patentblatt 88/4

(84) Benannte Vertragsstaaten:
AT BE CH DE ES FR GB GR IT LI LU NL SE

(71) Anmelder: PUMA Aktiengesellschaft Rudolf Dassler
Sport
Würzburger Strasse 13
D-8522 Herzogenaurach(DE)

(72) Erfinder: Gerhäuser, Heinz, Dr.-Ing.
Saugendorf 17
D-8552 Waischenfeld(DE)

(72) Erfinder: Pirner, Gerhard, Dipl.-Ing.
Lehenhammer 4
D-8459 Etzelwang(DE)

(72) Erfinder: Rückert, Thomas
Südstrasse 22
D-8551 Hausen(DE)

(74) Vertreter: Hufnagel, Walter, Dipl.-Ing.,
Dipl.-Wirtsch.-Ing. et al,
Dorner & Hufnagel Patentanwälte Bad Brückenauer Str.
19
D-8500 Nürnberg 90(DE)

(54) Verfahren zum Messen von Bewegungsabläufen bei Laufdisziplinen.

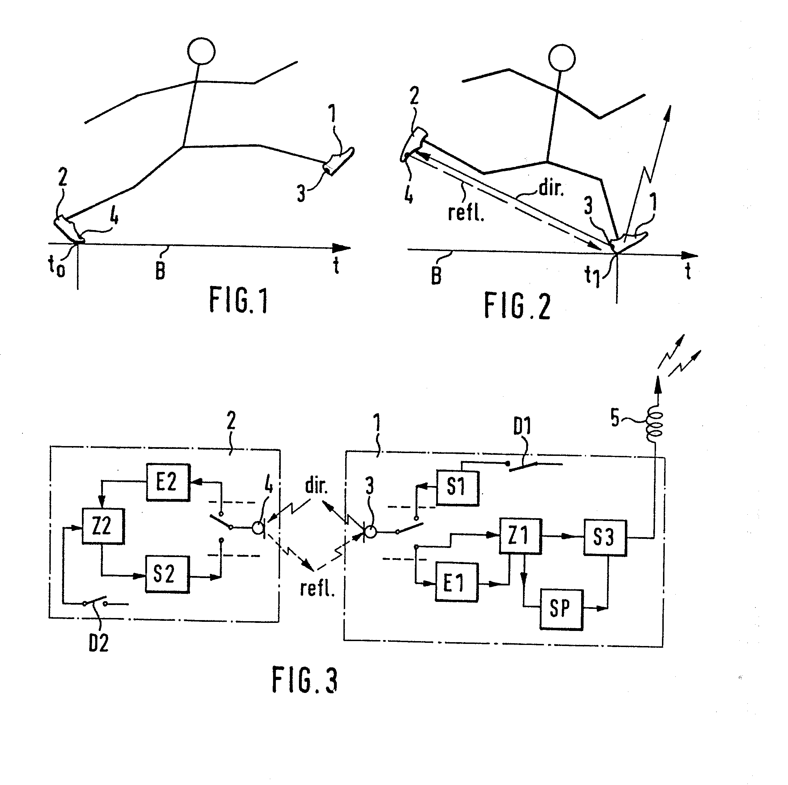

(57) Bei einem Verfahren zum Messen von Bewegungsabläufen bei Laufdisziplinen und daraus errechenbarer Größen, inbesondere der Schrittweite, Schrittzahl, Schrittzeit und Laufzeit, wird in jedem Schuh eines zugeordneten Schuhpaares (1, 2) ein Sender (S1 bzw. S2), ein Empfänger (E1 bzw. E2) und ein Signal-Laufzeit-Zähler (Z1 bzw. Z2) vorgesehen. Die vom hinteren Schuh (2) gemessene Laufzeit ($t_{dir.}$) eines vom vorderen Schuh (1) beim Auftreten abgegebenen Signals (dir.) wird zeitkonform auf den ersten Schuh (1) übertragen und es werden von diesem die Zählerstände beider Zähler (Z1 und Z2) an eine Rechnereinheit, insbesondere hochfrequent, abgestrahlt (Fig. 3).

FIG.3

EP 0 253 984 A1

Croydon Printing Company Ltd

PUMA Aktiengesellschaft
Rudolf Dassler Sport
8522 Herzogenaurach

_1_

Verfahren zum Messen von Bewegungsabläufen bei Laufdisziplinen

Die vorliegende Erfindung bezieht sich auf ein Verfahren zum Messen von Bewegungsabläufen bei Laufdisziplinen und daraus errechenbarer Größen gemäß dem Oberbegriff des Anspruches 1.

Ein derartiges Verfahren ist aus der DE-OS 34 05 081 bekannt. Bei diesem Verfahren wird vom vorderen Schuh beim Auftreffen auf dem Untergrund ein erstes Signal zum hinteren Schuh und zugleich ein Startsignal zu einer externen Rechnereinheit abgegeben. Vom hinteren Schuh wird darauf ein zweites Signal zum vorderen Schuh und über diesen ebenfalls zur Rechnereinheit gesendet. Aus den Laufzeiten der beiden Signale kann die Bein- oder Laufgeschwindigkeit und/oder die Schrittweite des Sportlers durch die Rechnereinheit ermittelt und ausgegeben werden.

Mit der vorliegenden Erfindung soll die Aufgabe gelöst werden, die Genauigkeit dieses bekannten Meßverfahrens zu erhöhen. Gleichzeitig soll ermöglicht werden, mit diesem Verfahren die Sprungzeit, sowie daraus ableitbare Größen zu messen.

Gelöst wird diese Aufgabe durch die im Kennzeichen des Anspruches 1 angegebenen Merkmale.

Durch die Verwendung von zwei Zählern in Verbindung mit den Sende-Empfangseinrichtungen kann auf einfache Weise sehr genau und ohne großen Bauteileaufwand die Sprungzeit und die Signallaufzeit gemessen werden.

Eine weitere Vereinfachung ist möglich durch Verwendung je eines einfachen Sende- Empfangs-Wandlers, der sowohl im Sendebetrieb als auch im Empfangsbetrieb wirksam geschaltet werden kann.

Weitere vorteilhafte Einzelheiten der Erfindung sind in den Unteransprüchen angegeben und werden nachfolgend anhand des in der Zeichnung dargestellten Ausführungsbeispieles näher beschrieben. Dabei zeigen:

Fig. 1 den Beginn der Sprungphase eines Läufers im Zeitpunkt des Abhebens des hinteren Fußes,

Fig. 2 das Ende der Sprungphase beim Aufsetzen des vorderen Fußes,

Fig. 3 das Prinzipschaltbild der beiden Sende-Empfangseinrichtungen und

Fig. 4 das dabei entstehende Ablaufschema.

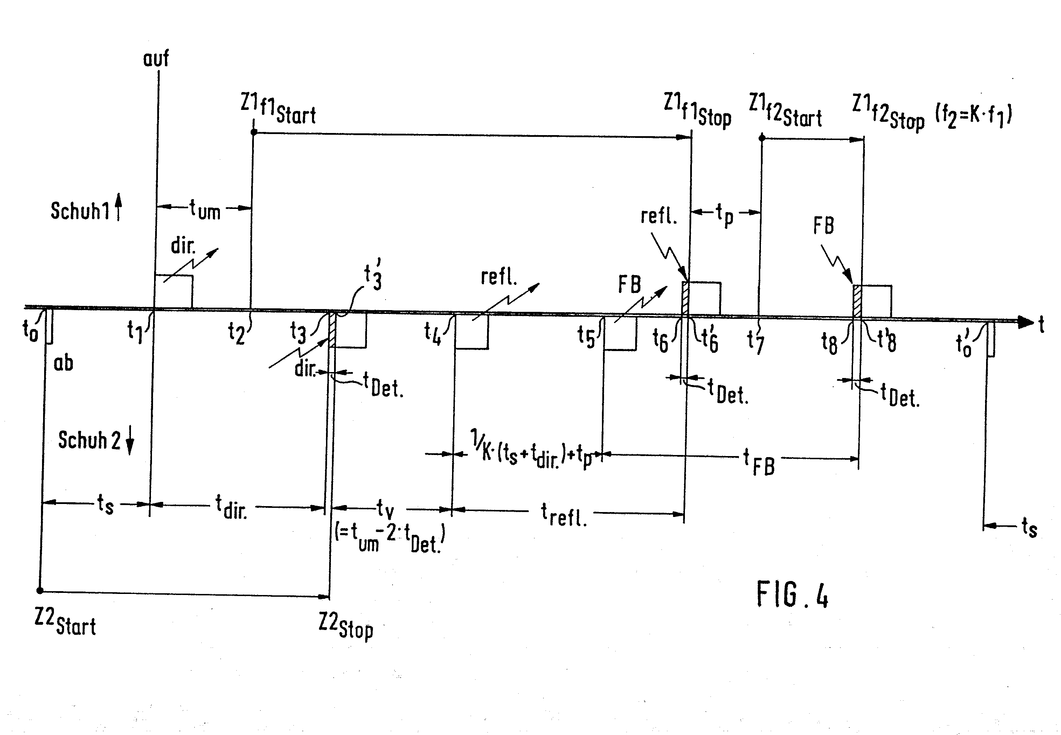

In Figur 4 sind auf der Zeitachse t nach oben die vom ersten Schuh 1 und nach unten die vom zweiten Schuh 2 abgegebenen und empfangenen Signale in den einzelnen Zeitabschnitten dargestellt. Dabei ist der erste Schuh 1 jeweils dem gerade vorn und der zweite Schuh 2 dem gerade hinten befindlichen Fuß zugeordnet, wie anhand der Fig. 1 und 2 dargestellt ist. In beiden Schuhen 1 und 2 ist je eine Sende- und eine Empfangseinheit S1, E1 bzw. S2, E2 (Fig. 3), insbesondere auf Ultraschallbasis, vorgesehen. Zweckmäßig ist dabei nur jeweils ein Wandler 3 bzw.

4 vorgesehen, der sowohl im Sendebetrieb als auch im Empfangsbetrieb arbeitet. Die Umschaltung erfolgt in der jeweiligen Sende- Empfangseinheit S1, E1 bzw. S2, E2. Vorteilhaft sind beide Sende- Empfangseinheiten S1, E1 bzw. S2, E2 gleichartig ausgeführt.

Zusätzlich ist im ersten Schuh 1 noch ein weiterer Sender S3 vorgesehen, der die im zweiten Schuh 2 vorhandenen und gegebenenfalls zwischengespeicherten Daten an eine externe Rechnereinheit abstrahlen kann.

In jedem Schuh 1, 2 ist noch ein Drucksensor D1 bzw. D2 vorgesehen. Der Drucksensor D1 des ersten Schuhes 1 bewirkt beim Auftreffen des Schuhes 1 die Ausgabe eines Ausgangssignals dir. (direkter Strahl) vom Sender S1 aus, das vom Wandler 4 dem Empfänger E2 zugeleitet wird. Nach Ausgabe des Ausgangssignals dir. wird an den Wandler 3 der Empfänger E1 angeschaltet und im Schuh 2 nach Empfang des Ausgangssignals dir. von Empfang auf Senden geschaltet und dann ein reflektiertes Ausgangssignal refl. an den ersten Schuh 1 ausgegeben und dort vom Wandler 3 und Empfänger E1 empfangen.

Zusätzlich enthält jeder Schuh 1, 2 noch einen Zähler und der erste Schuh 1 noch einen Speicher zur Speicherung mindestens eines von einem Zähler übernommenen Wertes.

Die Wirkungsweise dieser Anordnungen wird nachfolgend anhand des in Fig. 4 dargestellten Ablaufschemas erläutert.

Zum Zeitpunkt $t_o$ wird beim Laufen, bei dem eine Sprungphase auftritt, in der keiner der beiden Schuhe 1, 2 den Untergrund B berührt, beim Abheben des zweiten Schuhes 2 vom Untergrund B über den Drucksensor D2 der zweite Zähler Z2 gestartet.

Nach der Sprungzeit $t_s$ trifft der erste Schuh 1 zum Zeitpunkt $t_1$ auf dem Untergrund B auf. Dabei wird über dessen Drucksensor D1 dessen Sender S1 angesteuert und der direkte Strahl dir. als Ausgangssignal über den Wandler 3 zum hinteren zweiten Schuh 2 abgestrahlt. Nach Absendung des Ausgangssignals dir. wird innerhalb der Umschaltzeit $t_{um}$ der Wandler 3 auf den Empfänger E1 geschaltet und auf Empfangsbetrieb umgeschaltet und im Zeitpunkt $t_2$ der erste Zähler Z1 gestartet.

Nach der Laufzeit $t_{dir.}$ des Ausgangssignals dir. wird dieses vom Wandler 4 im Zeitpunkt $t_3$ aufgenommen und im Empfänger E2 verarbeitet, wobei das zu verarbeitende Signal nach einer Detektionszeit $t_{Det.}$, beispielsweise nach dem Einschwingvorgang der Empfangsschaltung, im Zeitpunkt $t_3'$ zur Verfügung steht. Dieses Signal wird zum Stoppen des zweiten Zählers Z2 herangezogen. Der Wert des Zählers Z2 entspricht daher der Sprungzeit $t_s$ zuzüglich der Laufzeit des direkten Strahles $t_{dir.}$ vom ersten Schuh 1 zum zweiten Schuh 2, zuzüglich der Detektionszeit $t_{Det.}$.

Nach einer Verzögerungszeit $t_v$ im zweiten Sende- Empfänger S2, E2 wird zum Zeitpunkt $t_4$ vom Sender S2 über den Wandler 4 ein reflektiertes Ausgangssignal refl. vom zweiten Schuh 2 an den ersten Schuh 1 abgestrahlt und von letzterem nach einer Laufzeit $t_{refl.}$ im Zeitpunkt $t_6$ empfangen. Das entsprechende Signal steht zum Zeitpunkt $t_6'$, also ebenfalls erst nach einer Detektionszeit $t_{Det.}$, zur Verfügung. Dieses Signal wird dazu herangezogen, den ersten Zähler Z1 zu stoppen.

Ist die Verzögerungszeit $t_v$ gleich der Umschaltzeit $t_{um}$ gewählt worden, dann entspricht der Wert des Zählers Z1 genau der Summe der Laufzeiten der Ausgangssignale dir. und refl., nämlich $t_{dir.} + t_{refl.}$.

Erfindungsgemäß wird nach Abgabe des reflektierten Ausgangssignales refl., also nach dem Zeitpunkt $t_4$, der Wert des zweiten Zählers Z2 an den Schuh 1 abgestrahlt. Dies erfolgt hier nach einer entsprechenden Zeitspanne von $t_4$ bis $t_5$, die der Sprungzeit $t_s$, zuzüglich der Laufzeit des direkten Ausgangssignals $t_{dir.}$, zuzüglich gegebenenfalls einer Pausenzeit $t_p$ entspricht, durch Abgabe eines Folge-Burst FB im Zeitpunkt $t_5$.

Die Pausenzeit $t_p$ ist dabei gleich der Pausenzeit $t_p$ im Zeitpunkt $t_7$, zu dem der erste Zähler Z1 nach seinem Stoppzustand im Zeitpunkt $t'_6$ wieder gestartet wird.

Der Folge-Burst FB wird nach einer Laufzeit $t_{FB}$ im Zeitpunkt $t_8$ vom Empfänger E1 empfangen, wobei das Verarbeitungssignal nach der Detektionszeit $t_{Det.}$ im Zeitpunkt $t'_8$, zur Verfügung steht. Zu diesem Zeitpunkt wird der erste Zähler Z1 durch den Folge-Burst FB gestoppt.

In der Pausenzeit $t_p$ wird der erste Wert des ersten Zählers Z1 in einem Speicher SP abgespeichert.

Ab dem Zeitpunkt $t'_6$ wird der weitere Sender S3 des ersten Schuhes 1 aktiviert und der im Speicher SP gespeicherte Wert und dann auch der zweite gezählte, der Zeit $t_{FB}$ entsprechende Wert über einen geeigneten Strahler 5 abgestrahlt und von einem externen Empfänger einer externen Rechnereinheit zugeleitet. Der Sender S3 ist vorteilhaft ein Sender für hochfrequente, elektromagnetische Wellen und der Strahler 5 eine hierfür geeignete Antenne.

Damit liegen an der Rechnereinheit und vorher schon im ersten Schuh 1 die Informationen über die doppelte Laufzeit des direkten Ausgangssignales dir. und die um die einfache Laufzeit $t_{dir.}$ zu große Sprungzeit $t_s$ vor. Aus

diesen Daten kann die tatsächliche Sprungzeit $t_s$ gewonnen werden, was zweckmäßig erst nach der Funkphase (Abstrahlung der Werte an die externe Rechnereinheit) im Mikroprozessor geschieht, da die dazu nötige Arithmetik dort keinen Aufwand verursacht.

Um die Übertragung der im zweiten Zähler Z2 gezählten Werte in der kurzen verfügbaren Zeit zu gewährleisten, erfolgt die Übertragung der Werte in einer um einen Faktor K verkürzten Zeit. Gleichzeitig wird der erste Zähler Z1 mit einer Frequenz $f_2$ angesteuert, die um den Faktor K größer ist als die vorher verwendete Frequenz $f_1$. Die Übertragungszeit zwischen den Zeitpunkten $t_4$ und $t_5$ beträgt demnach dann $\frac{1}{K} \cdot (t_s + t_{dir.}) + t_p$.

Geht der Anwender des erfindungsgemäßen Verfahrens vom Laufen in die Bewegungsart Gehen über, so entfällt die Sprungphase und damit die Sprungzeit $t_s$. Der Zeitpunkt $t_o$ liegt dann auf jeden Fall nach dem Zeitpunkt $t_1$. Der dann gestartete zweite Zähler Z2 wird in diesem Falle nicht vom direkten Ausgangssignal dir. gestoppt und läuft somit ab. Entsprechend wird keine Sprungzeit gezählt, auch kein Folge-Burst FB übertragen und damit kein Stoppsignal für den zum zweiten mal gestarteten Zähler Z1. Dadurch läuft der Zähler Z1 ebenfalls ab und es wird an die Rechnereinheit nur der beim ersten Start des Zählers Z1 ermittelte Wert $t_{dir.} + t_{refl.}$ übertragen. Damit ergibt sich aus den Laufzeiten $t_{dir.} + t_{refl.}$ bei gegebener Frequenz durch Umrechnung unmittelbar der doppelte Abstand der Schuhe 1 und 2 und damit die doppelte Schrittweite.

Um die Übertragungssicherheit zu erhöhen, ist es vorteilhaft, zumindest die vom ersten Schuh 1 an die externe Rechnereinheit abgestrahlten Signale in einem geeigneten Code zu übertragen, der es erlaubt, Übertragungsfehler zu erkennen und zu korrigieren. In gleicher Weise kann auch die am Schuh 2 gemessene Sprungzeit $t_s$ codiert auf den Schuh 1 übertragen werden.

------------------

PUMA Aktiengesellschaft
Rudolf Dassler Sport

8522 Herzogenaurach

P a t e n t a n s p r ü c h e

1. Verfahren zum Messen von Bewegungsabläufen bei
Laufdisziplinen und daraus errechenbarer Größen, insbesondere der Schrittweite, Schrittzahl, Schrittzeit und
Laufzeit, mit einem Laufschuhpaar, wobei in jedem Schuh
ein Sender und ein Empfänger vorgesehen ist und beim
Auftreffen des ersten Schuhs auf dem Untergrund dieser,
ausgelöst durch einen Drucksensor, ein Ausgangssignal
an den zweiten Schuh ausgibt und der Empfänger desselben
dieses empfängt und seinerseits über seinen Sender ein
Reflex-Ausgangssignal an den Empfänger des ersten Schuhs
ausgibt und der erste Schuh darauf ein zweites Ausgangssignal an einen externen Empfänger, an den eine Rechnereinheit angeschlossen ist, ausgibt und die Rechnereinheit aus der Laufzeit der Signale die gewünschten ermittelbaren Daten berechnet und ausgibt, dadurch gekennzeichnet,
daß beim Auftreffen des ersten Schuhes (1) zusätzlich ein
erster Zähler (Z1) gestartet wird, der beim oder nach
dem Empfang des Reflex-Ausgangssignales (refl.) gestoppt wird,
daß beim Abheben des zweiten Schuhes (2) über einen in
diesem eingebauten Sensor (D2) ein zweiter Zähler (Z2) gestartet wird, der beim oder eine definierte Zeit nach dem
Empfang des Ausgangssignales (dir.) des ersten Schuhes (1)
gestoppt wird, daß der gezählte Wert des zweiten Zählers (Z2)
an den ersten Schuh (1) abgestrahlt und von diesem empfangen

**0253984**

wird und daß aus den beiden Zählerständen (Z1 + Z2) die Schrittweite und/oder die Schrittzeit und/oder die Schrittzahl und/oder die Laufzeit und/oder die Sprungzeit ($t_s$) berechnet wird bzw. werden.

2. Verfahren nach Anspruch 1, <u>dadurch gekennzeichnet</u>, daß zumindest ein Teil der zu übertragenden Signale (dir., refl.) codiert übertragen wird.

3. Verfahren nach Anspruch 1 oder 2, <u>dadurch gekennzeichnet</u>, daß für jede Sende-.Empfangseinheit (S1, E1; S2, E2) jedes Schuhes (1, 2) ein einziger , in die beiden Betriebsarten umschaltbarer Sende-Empfangs-Wandler (3, 4) verwendet wird.

4. Verfahren nach Anspruch 3, <u>dadurch gekennzeichnet</u>, daß der erste Zähler (Z1) nach einer der Umschaltzeit ($t_{um}$) von Senden auf Empfang entsprechenden Verzögerungszeit gestartet wird und daß nach Empfang des Ausgangssignals (dir.) das Reflex-Ausgangssignal (refl.) um eine Verzögerungszeit ($t_v$) verzögert abgestrahlt wird, die wenigstens annähernd der Umschaltzeit ($t_{um}$) entspricht.

5. Verfahren nach Anspruch 4, <u>dadurch gekennzeichnet</u> daß die Verzögerungszeit ($t_v$) zur Abstrahlung des Reflex-Ausgangssignales (refl.) der Umschaltzeit ($t_{um}$) minus der Einschwingzeit ($t_{Det.}$) der Empfänger (E1, E2) des ersten und des zweiten Schuhes (1, 2) beträgt.

6. Verfahren nach einem der Ansprüche 1 bis 5, <u>dadurch gekennzeichnet</u>, daß nach einer Pausenzeit ($t_p = t_{um}$) nach dem Stoppen des ersten Zählers (Z1) dessen Wert abgespeichert und danach der erste Zähler (Z1) erneut gestartet wird und daß vom Sender (S2) des zweiten Schuhes (2) nach einer dem Zählerstand des zweiten Zählers (Z2) entsprechenden Zeit ($t_s + t_{dir.}$), zuzüglich der Pausenzeit ($t_p$) ein Stoppsignal (FB) an den ersten Schuh (1) ausgegeben wird, das den ersten Zähler (Z1) stoppt.

7. Verfahren nach Anspruch 6, dadurch gekennzeichnet, daß die zur Übertragung des Zählerstandes des zweiten Zählers (Z2) entsprechende Zeit ($t_s$ + $t_{dir.}$) um einen Faktor (K) verkürzt und die Zählfrequenz ($f_2$) des ersten Zählers (Z1) gegenüber der Zählfrequenz ($f_1$) bei der ersten Zählperiode um diesen Faktor (K) erhöht wird.

8. Verfahren nach einem der Ansprüche 1 bis 7, dadurch gekennzeichnet, daß die Sprungzeit ($t_s$), d.h. die Zeit während der kein Schuh (1, 2) den Untergrund (B) berührt, unter Berücksichtigung der Laufzeiten der Signale ($t_{dir.}$, $t_{refl.}$), der Verzögerungszeiten ($t_{um}$) und der Pausenzeit ($t_p$) in der Rechnereinheit berechnet und ausgegeben wird.

9. Verfahren nach einem der Ansprüche 1 bis 8, dadurch gekennzeichnet, daß die Sendesignale (dir., refl.) aus zwischen den beiden Schuhen (1, 2) ausgestrahlten Ultraschallimpulsen bestehen und als Wandler (3, 4) Ultraschallwandler verwendet werden.

10. Verfahren nach einem der Ansprüche 1 bis 9, dadurch gekennzeichnet, daß die vom ersten Schuh (1) zur externen Rechnereinheit ausgesandten Signale als hochfrequente, elektromagnetische Wellen übertragen werden.

-------------

0253984

**FIG.1**

**FIG.2**

**FIG.3**

FIG. 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A,D | EP-A-0 152 057 (PUMA-SPORTSCHUHFABRIKEN RUDOLF DASSLER KG) * Seite 8, Zeile 15 - Seite 11, Zeile 24; Figur 6 * | 1-3 | G 01 C 22/02 |
| A | EP-A-0 119 009 (NIKO INTERNATIONAL LTD.) * Zusammenfassung, Figur 1 * | 1 | |
| A | DE-A-3 514 130 (H.D. MUFENBACH) * Anspruch 1, Figuren 1, 2 * | 1 | |

RECHERCHIERTE SACHGEBIETE (Int Cl 4)

G 01 C 22/00
A 43 B 3/00

Der vorliegende Recherchenbericht wurde für alle Patentansprüche erstellt.

| Recherchenort | Abschlußdatum der Recherche | Prüfer |
|---|---|---|
| BERLIN | 23-11-1987 | WEIHS J.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein- stimmendes Dokument

EPA Form 1503 03 82